Europäisches Patentamt

European Patent Office

Office européen des brevets

(19)

(11) Veröffentlichungsnummer: **0 229 010 B1**

(12) # EUROPÄISCHE PATENTSCHRIFT

(45) Veröffentlichungstag der Patentschrift :
**10.04.91 Patentblatt 91/15**

(51) Int. Cl.$^5$: **A61K 6/00**

(21) Anmeldenummer : **86890340.2**

(22) Anmeldetag : **16.12.86**

(54) **Haftmittel für Zahnprothesen od.dgl. und Verfahren zu seiner Herstellung.**

(30) Priorität : **31.12.85 DE 3546367**

(43) Veröffentlichungstag der Anmeldung :
**15.07.87 Patentblatt 87/29**

(45) Bekanntmachung des Hinweises auf die
Patenterteilung :
**10.04.91 Patentblatt 91/15**

(84) Benannte Vertragsstaaten :
**AT BE CH DE ES FR GB GR IT LI LU NL SE**

(56) Entgegenhaltungen :
**AT-A- 368 694**
**DE-A- 2 924 095**
**FR-A- 2 036 382**
**FR-A- 2 072 432**

(73) Patentinhaber : **Altwirth, Oskar**
**Oberach 37**
**A-4950 Altheim (AT)**

(72) Erfinder : **Altwirth, Oskar**
**Oberach 37**
**A-4950 Altheim (AT)**

(74) Vertreter : **Hübscher, Gerhard, Dipl.-Ing. et al**
**Patentanwälte Dipl.-Ing. Gerhard Hübscher**
**Dipl.-Ing. Helmut Hübscher Dipl.-Ing. Heiner**
**Hübscher Spittelwiese 7**
**A-4020 Linz (AT)**

EP 0 229 010 B1

**Beschreibung**

Die Erfindung bezieht sich auf ein Haftmittel für Zahnprothesen, aus einer streichfähigen, ein Alginat und ein Polyvinylacetat enthaltenden Masse sowie auf ein Verfahren zum Herstellen eines solchen Haftmittels.

Um ein streichfähiges, in Tuben abfüllbares Haftmittel zu gewinnen, wurde gemäß der DE-OS 20 56 363 des Anmelders bereits vorgeschlagen, ein Alginat mit einer alkoholischen Lösung eines physiologisch einwandfreien Festharzes, vorzugsweise Polyvinylacetat, zu mischen, doch hat sich dieses Haftmittel in der Praxis nicht bewährt, da es schon kurzzeitig nach der Tubenabfüllung zu einer Entmischung von Alginat und Polyvinylacetat und dadurch zu einem Wirkungsverlust des Haftmittels kam. Außerdem veränderte sich, bedingt durch den Abgang von Alkohol auf Grund vorhandener Undichtheiten der Tubenverschlüsse, die Viskosität des Haftmittels, das damit seine Streichfähigkeit verlor, und zusätzlich ging im Lauf der Zeit das Alginat selbst eine starke Bindung mit dem Polyvinylacetat ein, so daß die Aufgabe des Alginates, in Flüssigkeit aufzuquellen und Feuchtigkeit aufzunehmen, um die Adhäsion zu erhöhen, nur mehr unzureichend erfüllt wurde.

Andere bekannte Haftmittel, die ein Alginat enthalten, sind pulverförmig, was das gleichmäßige Auftragen erschwert und wegen der Abspülmöglichkeit durch die Speise- und Getränkeaufnahme nur mit kurzer Wirkungsdauer verbunden ist. Ähnliches gilt für ebenfalls bekannte Haftmittel, bei denen ein Alginat in einer pastenförmigen Trägersubstanz, vorzugsweise Vaseline, eingemengt wird, da auch diese Trägersubstanz samt dem Alginat von der Prothese weggeschwemmt wird.

Der Erfindung liegt daher die Aufgabe zugrunde, diese Mängel zu beseitigen und ein Haftmittel der eingangs geschilderten Art zu schaffen, das sich durch seine langzeitige Haltbarkeit, seine bleibende Streichfähigkeit und nicht zuletzt durch seine gute und dauerhafte Klebefähigkeit auszeichnet.

Die Erfindung löst diese Aufgabe dadurch, daß die das Haftmittel bildende Masse aus einer Mischung des Alginates und des Polyvinylacetates mit Carboxymethylcellulose, einem Methylcellulose-Emulgator und einem Neutralöl-Weichmacher sowie einem alkoholischen Lösungsmittel, vorzugsweise 85%-iger Äthylalkohol besteht, wobei günstigerweise die Mischung gleiche Anteile an Alginat und Carboxymethylcellulose enthält. Durch die Einbettung des Alginates in Polyvinylacetat wird ein Abspülen bzw. Wegschwemmen des Alginates verhindert, wobei durch die Mundwärme das Polyvinylacetat erweicht und klebrig wird, so daß die Prothese an der vom die Feuchtigkeit der Schleimhaut aufnehmenden Alginat getrockneten Zahnfleischunterlage fest und dauerhaft klebt. Die starke Beeinträchtigung der Feuchtigkeitsaufnahmefähigkeit des Alginates durch das Polyvinylacetat wird durch die Beigabe von Carboxymethylcellulose, also mit Cellulosemolekülen, in die Glucoseeinheiten verknüpft sind, wieder aufgehoben und das Alginat erhält praktisch seine volle Fähigkeit der Feuchtigkeitsaufnahme zurück. Der Emulgator, beispielsweise ein Methylcellulose-Emulgator mit dem Firmennamen Tylopur MHB 50, verhindert eine Entmischung von Alginat und Polyvinylacetat und der Weichmacher, beispielsweise ein Neutralöl mit dem Handelsnamen Miglyol, ein Ester mittelkettiger Fettsäuren, oder Petrolatum, wie Vaseline, verleiht dem Polyvinylacetat auch nach Abgabe von Alkohol eine streichfähige Konsistenz, erlaubt das Abfüllen der pastenförmigen Mischung in handelsüblichen Tuben ohne Spezialverschluß und gewährleistet vor allem auch die einfache Reinigung der Prothesen.

Zur Herstellung des erfindungsgemäßen Haftmittels werden zunächst eine erste Mischung aus fein zerteiltem Polyvinylacetat und dem Weichmacher unter Alkoholzusatz und eine zweite Mischung aus einer alkoholischen Emulsion eines Gemenges von Alginat und Carboxymenthylcellulose und dem Emulgator hergestellt und dann beide Mischungen zusammengemengt und zu einer pastenartigen Masse geknetet.

Dabei können bei der Herstellung der ersten Mischung 20 Mengenteile Polyvinylacetat mit 80 Mengenteilen Äthylalkohol vermischt und dann 3 Mengenteile Weichmacher zugemengt werden, welcher Weichmacher vor dem Zumengen mit Äthylalkohol verdünnbar ist, und bei der Herstellung der zweiten Mischung 80 Mengenteilen eines zu gleichen Teilen aus Alginat und Carboxymethylcellulose zusammengemischten Pulvergemenges 20 Mengenteile Äthylalkohol zugemischt und in die entstehende, auf ca. 35°C erwärmte Emulsion 4 Mengenteile Emulgator eingerührt werden.

Bei der Haftmittelherstellung müssen daher zuerst die beiden Ausgangsmischungen vorbereitet werden, wobei für die Zubereitung der ersten Mischung Polyvinylacetat feinst, fast pulverförmig zu zerteilen und in 85%-igem Äthylalkohol zu lösen ist. Der entstehenden Emulsion wird in einer entsprechenden Misch- und Knetmaschine der Weichmacher zugemengt, bis in der Mischung etwa 3% Weichmacher enthalten sind. Um die Vermengung zu erleichtern, kann der Weichmacher vor dem Zusetzen mit Äthylalkohol verdünnt werden. Zur Vorbereitung der zweiten Mischung wird in einem gesonderten Rührwerk, und zwar in einem Vakuummischer, Alginat zu gleichen Teilen mit Carboxymethylcellulose vermengt, welche Carboxylmethylcellulose, die ein Polyelectroloid mit anionogenem Charakter ist, gut mit anderen anionogenen Produkten, wie Alginat, Polyvinylacetat und deren Mischungen bzw. Emulsionen, vermengt werden kann. Zur Verbesserung der Mischbedingung wird Äthylalkohol zugesetzt und die entstehende zähe Emulsion auf ungefähr 35°C erwärmt, um den Emulgator beizumengen. Dieser Emulgator, eine Methylcellulose-Verbindung, wird mittels Schnellrührers in die erwärmte Emulsion eingebracht, wobei intensives Rühren notwendig ist und die Beimengung in kleinen Mengen erfolgt, bis die zweite Mischung etwa 4% Emulgator enthält. Nach dem Abkühlen der zweiten Mischung werden dann beide Mischungen, also das Gemisch Polyvinylacetat mit Weichmacher und das Gemisch Alginat mit Carboxymethylcellulose und Emulgator in einer eigenen Knetmaschine miteinander vermischt, bis die gewünschte homogene, zähe, pastenartige Masse entsteht, die anschließend in Tuben abgefüllt werden kann.

Um diese Masse zu erhalten, sind vorzugsweise 100 Mengenteile der ersten und 70 Mengenteile der zweiten Mischung zusammenzumengen, wobei der Misch und Knetvorgang solange durchgeführt wird, bis durch entsprechenden Alkoholverlust, d.i. ca. 13% der Mischung, die gewünschte Konsistenz erreicht ist. Dazu wird ein eigenes Rührwerk verwendet, das mit einer elektronisch gesteuerten Waage die genau vorherbestimmbare Gewichtsabnahme durch den Verlust an Äthylalkohol anzeigt.

## Ansprüche

1. Haftmittel für Zahnprothesen, aus einer streichfähigen, ein Alginat und ein Polyvinylacetat enthaltenden Masse, dadurch gekennzeichnet, daß die Masse aus einer Mischung des Alginates und des Polyvinylacetates mit Carboxymethylcellulose, einem Methylcellulose-Emulgator und einem Neutralöl-Weichmacher sowie einem alkoholischen Lösungsmittel, vorzugsweise 85%-iger Äthylalkohol, besteht.

2. Haftmittel nach Anspruch 1, dadurch gekennzeichnet, daß die Mischung gleiche Anteile an Alginat und Carboxymethylcellulose enthält.

3. Verfahren zum Herstellen eines Haftmittels nach Anspruch 1 oder 2, dadurch gekennzeichnet, daß zunächst eine erste Mischung aus feinzerteiltem Polyvinylacetat und dem Weichmacher unter Alkoholzusatz und eine zweite Mischung aus einer alkoholischen Emulsion eines Gemenges von Alginat und Carboxymethylcellulose und dem Emulgator hergestellt und dann beide Mischungen zusammengemengt und zu einer pastenartigen Masse geknetet werden.

4. Verfahren nach Anspruch 3, dadurch gekennzeichnet, daß bei der Herstellung der ersten Mischung 20 Mengenteile Polyvinylacetat mit 80 Mengenteilen Äthylalkohol vermischt und dann 3 Mengenteile Weichmacher zugemengt werden, wobei vorzugsweise der Weichmacher vor dem Zumengen mit Äthylalkohol verdünnt wird.

5. Verfahren nach Anspruch 3, dadurch gekennzeichnet, daß bei der Herstellung der zweiten Mischung 80 Mengenteilen eines zu gleichen Teilen aus Alginat und Carboxymethylcellulose zusammengemischten Pulvergemenges 20 Mengenteile Äthylalkohol zugemischt und in die entstehende, auf ca. 35°C erwärmte Emulsion 4 Mengenteile Emulgator eingerührt werden.

6. Verfahren nach einem der Ansprüche 3 bis 5, dadurch gekennzeichnet, daß 100 Mengenteile der ersten Mischung mit 70 Mengenteilen der zweiten Mischung vermengt werden.

## Claims

1. An adhesive for dental prostheses, consisting of a spreadable substance containing an alginate and a polyvinyl acetate, characterised in that the substance consists of a mixture of the alginate and of the polyvinyl acetate with carboxymethyl cellulose, a methyl cellulose emulsifier and a neutral oil plasticiser and an alcoholic solvent, preferably 85% ethyl alcohol.

2. An adhesive according to claim 1, characterised in that the mixture contains equal proportions of alginate and carboxymethyl cellulose.

3. A process for the preparation of an adhesive according to claim 1 or 2, characterised in that a first mixture of finely divided polyvinyl acetate and the plasticiser with an addition of alcohol is first made and then a second mixture is made of an alcoholic emulsion of a mixture of alginate and carboxymethyl cellulose and the emulsifier, and then the two mixtures are mixed together and kneaded to form a pasty substance.

4. A process according to claim 3, characterised in that in the production of the first mixture 20 parts by quantity of polyvinyl acetate are mixed with 80 parts by quantity of ethyl alcohol and then 3 parts by quantity of plasticiser are added, the plasticiser preferably being diluted before mixing with ethyl alcohol.

5. A process according to claim 3, characterised in that in the production of the second mixture 20 parts by quantity of ethyl alcohol are added to 80 parts by quantity of a powder mixture prepared from equal parts of alginate and carboxymethyl cellulose, and 4 parts by quantity of emulsifer are stirred into the resulting emulsion heated to about 35°C.

6. A process according to any one of claims 3 to 5, characterised in that 100 part by quantity of the first mixture are mixed with 70 parts by quantity of the second mixture.

## Revendications

1. Adhésif pour prothèses dentaires, composé d'une pâte pouvant s'étaler et qui contient un alginate et un poly-acétate de vinyle, caractérisé par le fait que la pâte est composée d'un mélange formé de l'alginate et du poly-acétate de vinyle avec de la carboxy-méthyl-cellulose, d'un émulsifiant à base de méthyl-cellulose et d'un plastifiant à base d'une huile neutre, ainsi que d'un solvant alcoolique, de préférence d'alcool éthylique à 85%.

2. Adhésif selon la revendication 1, caractérisé par le fait que le mélange contient des proportions égales d'alginate et de carboxy-méthyl-cellulose.

3. Procédé de fabrication d'un adhésif selon la revendication 1 ou 2, caractérisé par le fait que l'on prépare tout d'abord un premier mélange de poly-acétate de vinyle finement divisé et du plastifiant avec addition d'alcool et un deuxième mélange composé d'une émulsion alcoolique d'un mélange d'alginate et de carboxy-méthyl-cellulose et de l'émulsifiant, et qu'on rassemble les deux mélanges et qu'on les malaxe pour former une masse pâteuse.

4. Procédé selon la revendication 3, caractérisé par le fait que, dans la préparation du premier mélange, on ajoute à 20 parties de poly-acétate de vinyle, 80 parties d'alcool éthylique et qu'on ajoute ensuite 3 parties de plastifiant, le plastifiant étant de préférence dilué avec de l'alcool éthylique avant d'être ajouté.

5. Procédé selon la revendication 3, caractérisé par le fait que, dans la préparation du deuxième mélange, on ajoute 20 parties d'alcool éthylique à 80 parties d'une poudre composite constituée à parties égales d'alginate et de carboxy-méthyl-cellulose et que l'on incorpore 4 parties d'émulsifiant par agitation dans l'émulsion ainsi obtenue, portée à 35°C environ.

6. Procédé selon l'une des revendications 3 à 5, caractérisé par le fait que l'on assemble 100 parties du premier mélange et 70 parties du deuxième mélange.